# FASCICULE DE BREVET EUROPEEN

(11) **EP 0 567 705 B1**
(45) Date de publication et mention de la délivrance du brevet: **09.07.1997**
(21) Numéro de dépôt: 92420144.5
(22) Date de dépôt: 30.04.1992
(51) Int. Cl.: A61F 2/38

(54) **Prothèse totale postéro-stabilisée du genou**
Totale von hinten stabilisierte Knieprothese
Posteriorly stabilized total knee prosthesis

(43) Date de publication de la demande: 03.11.1993
(73) Titulaire: MERCK BIOMATERIAL FRANCE, 01800 Charnoz (FR); Collomb, Jean, F-26800 Portes Les Valence (FR)
(72) Inventeur: Augoyard, Marc, F-69005 Lyon (FR); Bascoulergue, Gérard, F-62520 Le Touquet (FR); Basso, Maurice, F-83400 Hyères (FR); Berthocchi, René, 69006 Lyon (FR); Charret, Philippe, F-69270 Fontaine/Saône (FR); Courcelles, Philippe, F-69570 Dardilly (FR); Debiesse, Jean-Louis, F-38200 Vienne (FR); Eyraud, Guy, F-38150 Péage de Roussillon (FR); Fayard, Jean-Philippe, F-42170 St Just St Rambert (FR); Melere, Gilles, F-74000 Annecy (FR); Millon, Joseph, F-74490 La Ravoire (FR); Noyer, Daniel, F-38300 Maubec (FR); Passot, Jean-Paul, F-42530 St Genest Lerpt (FR); Peyrot, Jacques, F-69005 Lyon (FR); Pintore, Ernesto, I-84100 Salerno (IT); Relave, Marc, F-42580 L'Etrat (FR); Collomb, Jean, F-26000 Porte les Valence (FR); Majou, Claude, F-01800 Meximieux (FR); Lecuire, Francois, F-83400 Hyères (FR)
(74) Mandataire: Thibault, Jean-Marc

(56) Documents cités:
- EP-A- 0 294 298
- US-A- 4 892 547

## Description

La présente invention est relative aux prothèses du genou et elle vise, plus particulièrement, le domaine des prothèses totales.

Par prothèse totale, il convient de retenir les systèmes articulaires artificiels visant à remplacer l'articulation naturelle constituée par la conformation épiphysaire basse du fémur, par la conformation épiphysaire complémentaire haute du tibia et par l'élément fémoro-patellaire.

Il peut être considéré que, dans le domaine technique ci-dessus, les propositions connues de la technique antérieure peuvent se regrouper en deux grandes familles qui, de façon arbitraire, peuvent être dénommées, respectivement, celle des prothèses liées et celle des prothèses libres.

Par prothèses liées, il convient de retenir celles faisant intervenir deux pièces complémentaires qui sont réunies par un système articulaire matériel, tel qu'au moins un axe, constituant le système de pivotement artificiel, matérialisant l'articulation du genou, selon une direction perpendiculaire au plan sagittal ou antéro-postérieur.

La seconde famille, dite libre, est celle comprenant les prothèses constituées à base de deux éléments, respectivement adaptables sur les épiphyses basse du fémur et haute du tibia, pour coopérer, par glissement relatif, en étant maintenus en contact de surface par l'intermédiaire, notamment, des ligaments latéraux interne et externe naturels, sans présence de liens d'articulation entre elles.

L'invention concerne, uniquement, le domaine des prothèses dites libres.

Dans le domaine technique que vise l'objet de l'invention, on a proposé, depuis déjà quelques temps, de constituer des prothèses comprenant un élément fémoral adaptable après résection osseuse sur l'extrémité épiphysaire basse du fémur et présentant une partie antérieure délimitant une trochlée fémorale à partir de laquelle s'étendent deux condyles séparés présentant une courbure variable en direction de la face arrière.

Une prothèse libre comprend, par ailleurs, pour coopérer avec l'élément fémoral, un élément tibial adaptable après résection sur l'extrémité épiphysaire haute du tibia. Un tel élément tibial offre, en surface supérieure, un patin présentant deux cavités glénoïdes de coopération pour les condyles et une sorte d'arête centrale parallèle au plan sagittal, destinée à être insérée dans l'espace intercondylien de l'élément fémoral.

Une prothèse du genre ci-dessus laisse subsister, entre les condyles séparés, un espace libre permettant le passage des ligaments croisés qui sont responsables, pour l'essentiel, de la tenue du tibia par rapport au fémur dans le plan antéro-postérieur, dans un état de flexion notamment.

Dans certains cas particuliers, l'implantation d'une prothèse totale du genou implique de supprimer les ligaments croisés, en raison de leur déficience, de leur désinsertion ou de leur rupture.

Une prothèse totale du type ci-dessus présente alors, lorsqu'elle est implantée dans un tel cas, un inconvénient fonctionnel fondamental dans les mouvements faisant intervenir une flexion, par exemple dans le cas de montée ou de descente d'un escalier ou, encore, dans le cas de passage d'une position assise à une position debout du sujet.

En effet, en l'absence des ligaments croisés, une prothèse du type ci-dessus n'est pas de nature à s'opposer aux mouvements de tiroir du tibia par rapport au fémur, correspondant à un glissement, généralement vers l'arrière, des cavités glénoïdes du tibia par rapport aux condyles. Un tel mouvement de tiroir est traumatisant pour les ligaments latéraux, induit des usures ponctuelles du patin tibial et introduit un facteur de déséquilibre pour le sujet.

Afin de remédier à l'inconvénient ci-dessus, la technique antérieure a vu naître des prothèses totales libres, dites de postéro-stabilisation, en ce sens qu'elles sont conçues pour réintroduire, en l'absence des ligaments croisés, un blocage postérieur s'opposant à l'effet de tiroir.

De telles prothèses, qui peuvent être illustrées par les enseignements des brevets français **2 473 876** (**81-01 056**) et **2 615 386** (**87-07 353**), mettent à profit l'espace intercondylien de l'élément fémoral et comportent, à partir du plateau tibial, une saillie médiane destinée à s'engager dans l'espace intercondylien pour constituer, par sa face arrière, une surface d'appui, généralement à caractère concave.

L'élément fémoral comporte, dans la partie arrière de l'espace intercondylien, une butée de forme générale convexe qui se trouve suffisamment éloignée en position debout pour ne pas coopérer avec la saillie du plateau tibial contre laquelle cette butée est amenée en appui pour une flexion de l'ordre de 60^{o} environ.

Dans cette position, la butée coopère avec la surface d'appui pour centrer relativement les deux éléments de la prothèse en cours de flexion et s'opposer au glissement relatif responsable de l'effet de tiroir. De telles prothèses sont dénommées à fonction de stabilisation postérieure, en ce sens que les éléments constitutifs trouvent mutuellement un appui relatif arrière lors du déplacement relatif entre le tibia et le fémur.

Si la fonction de stabilisation postérieure en flexion est, de la sorte, assumée, il est important de noter que les structures préconisées aboutissent à réaliser des prothèses à caractère guidé, sensiblement parallèlement au plan sagittal, en raison de la pénétration de la saillie du plateau tibial dans l'espace intercondylien de l'élément fémoral.

Une telle structure se traduit, alors, par une liaison imposée du tibia par rapport au fémur selon le plan sagittal avec une simple possibilité de pivotement relatif sur un axe fictif qui peut être considéré comme étant généralement perpendiculaire au plan sagittal.

Une telle limitation de mouvement relatif à un seul degré de liberté n'est, en fait, pas compatible avec la caractéristique anatomique du genou naturel qui autorise une rotation relative du tibia par rapport au fémur, avec possibilité d'orientation du pied en rotation interne ou externe sur des plages angulaires, certes limitées, mais néanmoins nécessaires.

Des prothèses du type ci-dessus interdisent, au contraire, une telle rotation relative, ce qui se traduit par des contraintes importantes qui sont imposées, soit aux éléments en contact et en glissement de la prothèse totale, soit au moyen d'ancrage des éléments de la prothèse sur les épiphyses en vis-à-vis du fémur et du tibia.

De telles contraintes sont responsables de douleurs ou de descellement des éléments prothétiques qui doivent faire l'objet d'interventions de consolidation ou de démantellement ou de remembrement.

La présente invention vise à remédier aux inconvénients ci-dessus en proposant une nouvelle prothèse totale libre postéro-stabilisée du genou, dont la conception est justement prévue pour autoriser, en position postéro-stabilisée de flexion de l'articulation, une possibilité de rotation relative du tibia par rapport au genou, sans supprimer les contacts d'appui fondamentaux devant être maintenus entre les condyles fémoraux et les cavités glénoïdes tibiales.

Pour atteindre les objectifs ci-dessus, la prothèse selon l'invention est caractérisée en ce que :
- la butée est constituée par une éminence formée en saillie par la partie arrière du voile intercondylien et comprenant une tête antérieure prolongée vers l'arrière par un corps allongé sensiblement en forme de segment semi-torique se développant sensiblement dans le plan sagittal de l'élément,
- et la surface d'appui est définie par une cavité :
   . ménagée à partir du somment d'une épine séparant les cavités glénoïdes depuis le bord antérieur du patin,
   . définie par un rayon de courbure dans le plan sagittal supérieur à celui du segment semi-torique et par un rayon de courbure transversal supérieur à celui de la section transversale dudit segment,
   . s'ouvrant vers l'arrière et se raccordant à un sillon à flancs divergents aboutissant au bord arrière du patin.

Diverses autres caractéristiques ressortent de la description faite ci-dessous en référence aux dessins annexés qui montrent, à titre d'exemple non limitatif, une forme de réalisation de l'objet de l'invention.

La **fig. 1** est une coupe-élévation prise selon le plan sagittal de la prothèse selon l'invention considérée dans un état d'implantation réel entre le fémur et le tibia d'un sujet.

Les **fig. 2** et **3** sont des vues transversales prises, respectivement, selon les lignes **II-II** et **III-III** de la **fig. 1**.

Les **fig. 4** et **5** sont des perspectives de l'élément de prothèse fémorale permettant de mieux apprécier la structure de cet élément.

La **fig. 6** est une perspective mettant en évidence la structure de l'élément tibial de la prothèse.

La **fig. 7** est une coupe-élévation, analogue à la **fig. 1**, illustrant la prothèse totale dans une position particulière de flexion.

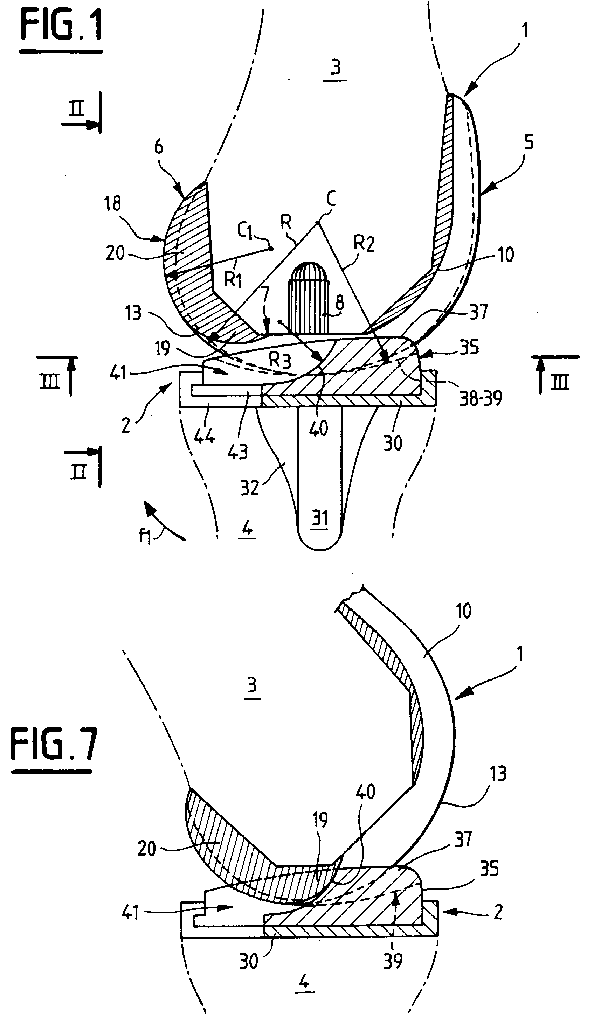

La **fig. 1** montre la prothèse totale selon l'invention constituée par un élément fémoral **1** et par un élément tibial **2**, destinés à être adaptés, respectivement, après résection osseuse, sur l'épiphyse basse **3** fémorale et l'épiphyse haute **4** tibiale.

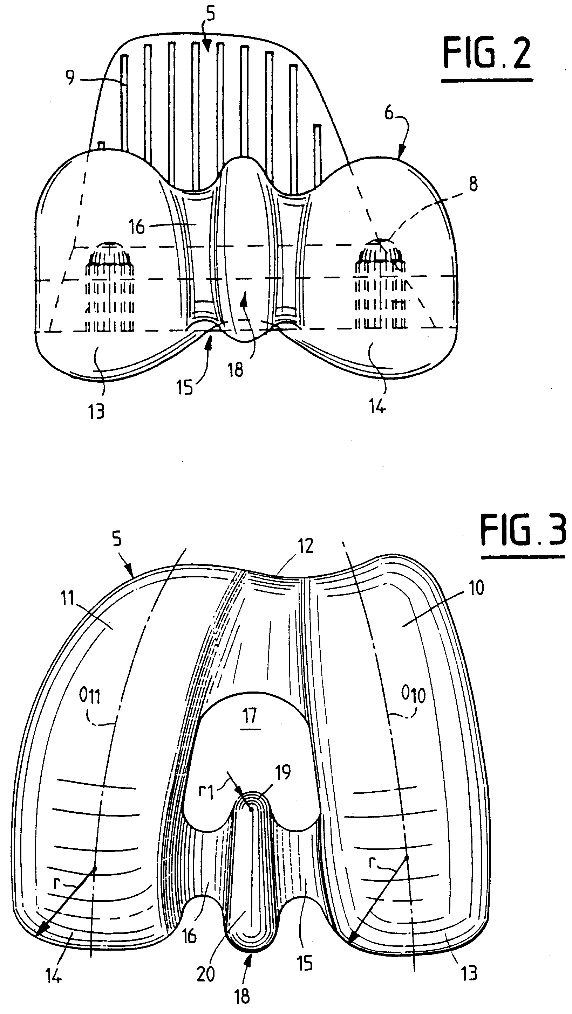

L'élément fémoral **1**, plus particulièrement illustré par les **fig. 2 à 5**, est réalisé en métal de préférence et, par exemple, en un alliage chrome-cobalt, bien connu dans le domaine technique considéré.

L'élément fémoral **1**, considéré selon une vue latérale qui serait parallèle au plan sagittal de l'ensemble fémur **3**-tibia **4** et, par exemple, matérialisé par la ligne **P-P'** à la **fig. 2**, présente, comme cela est illustré par la **fig. 1**, une forme sensiblement en **"U"** comprenant une branche **5**, dite avant ou antérieure, une branche **6**, dite arrière ou postérieure de plus faible longueur, et une âme **7** de liaison.

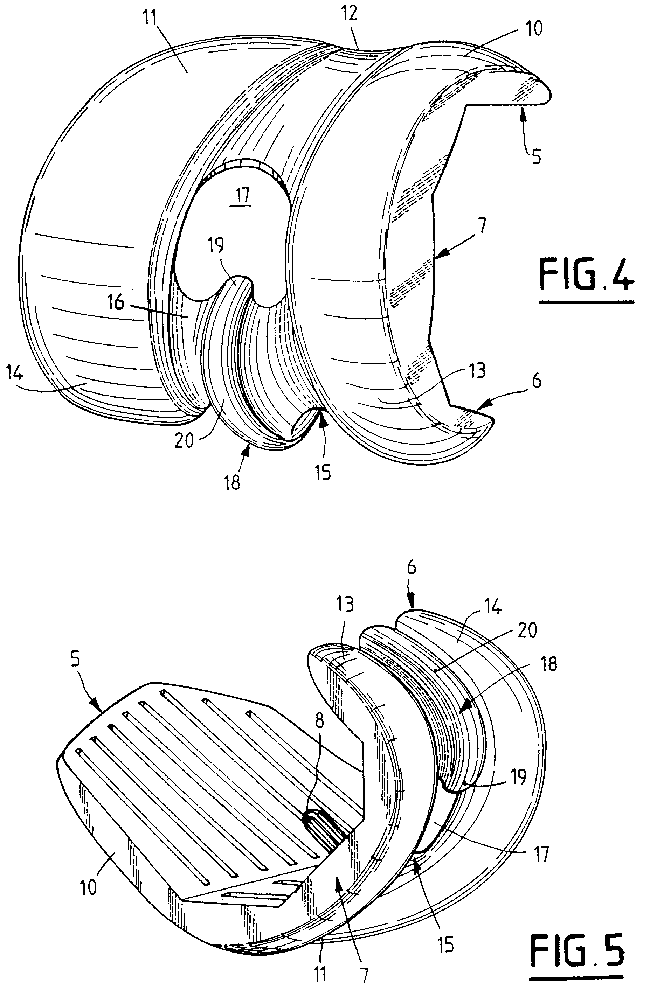

La surface interne, délimitée par l'élément fémoral, définit, en quelque sorte, un logement polygonal destiné à emboîter l'épiphyse **3** soumise, préalablement, à une résection articulaire complémentaire. L'adaptation et la liaison de l'élément fémoral sur l'épiphyse **3** est assurée, notamment, par l'intermédiaire de pions **8** pénétrant des trous borgnes dans l'épiphyse **3**, avec ou sans présence de ciment de liaison. Pour favoriser la repousse osseuse et la liaison interfacielle, les faces internes des branches **5, 6** et de l'âme **7** peuvent présenter un état de surface approprié faisant apparaître un grenaillage, des aspérités, des accidents, des picots, etc, tels que les accidents de surface portant la référence **9** aux **fig. 2** et **5**.

L'élément fémoral **1** est conformé pour présenter, par sa surface extérieure et en relation avec la branche **5**, une surface rotulienne définie par deux surfaces latérales **10** et **11** réuni es par une rainure **12** sensiblement verticale, délimitant une trochlée fémorale apte à coopérer avec la protubérance naturelle ou avec un bouton artificiel à même fonction présenté ou porté par la rotule, non représentée. Les deux surfaces latérales **10** et **11** affectent, transversalement au plan sagittal, des convexités évolutives et présentent, entre elles, un caractère divergent à partir de l'extrémité supérieure de la branche **5**, selon des orientations **O**_{**10**} et **O**_{**11**} matérialisées en trait mixte, en particulier à la **fig. 3**.

L'élément fémoral **1** forme, en surface extérieure et dans sa partie correspondant à l'âme **7** et à la branche **6**, deux condyles **13** et **14**, respectivement dits intérieur et extérieur, qui font suite aux surfaces latérales **10** et **11** divergentes, pour s'étendre ensuite de façon sensiblement parallèle, au moins à partir du milieu de l'âme **7** et le long de la branche **6**. Les condyles **13** et **14** sont définis par un rayon de courbure évolutif croissant d'arrière en avant et matérialisé aux dessins, pour des raisons de commodité uniquement, par le vecteur **R** de la **fig. 1**, centré sur le centre **C**.

Les condyles **13** et **14** sont définis, transversalement au plan sagittal, par des rayons de courbure **r** qui sont, également, variables et différents d'un condyle à l'autre, le condyle externe étant transversalement plus bombé que le condyle interne, tel que cela apparaît aux dessins.

Les condyles **13** et **14** définissent entre eux une fosse intercondylienne qui est occupée par un voile intercondylien **16** interrompu sensiblement dans la partie de l'âme de liaison **7** pour délimiter une fenêtre **17** destinée à réduire la résection osseuse.

Le voile intercondylien **16** forme, à partir du bord postérieur de la fenêtre **17** et sensiblement sur la longueur de la branche arrière ou postérieure **6**, une éminence **18** saillant dans la fosse intercondylienne **15** et se développant vers l'arrière en étant sensiblement centrée sur le plan **P-P'** sagittal. L'éminence **18** comporte une tête antérieure **19** qui est prolongée, vers l'arrière, par un corps allongé **20** sensiblement en forme de segment semi-torique dont le rayon **r**_{**1**} de la section transversale est, par exemple, de l'ordre du sixième de l'étendue transversale de la fosse intercondylienne **15**. La tête **19** présente donc une forme pouvant être déduite comme sensiblement semi-sphérique. Le corps sensiblement semi-torique **20** est défini par un rayon de courbure **R**_{**1**}, inférieur au rayon **R**, centré sur un centre **C**_{**1**} qui est tel que, de préférence, le pourtour extérieur du corps **20** s'étende à l'extérieur de l'enveloppe matérialisée par les condyles **13** et **14**.

Selon l'invention, l'élément tibial **2** comprend un plateau **30** dont la face inférieure est pourvue d'au moins une queue **31** éventuellement renforcée par des goussets **32**. La queue **31** est destinée à être implantée dans l'épiphyse **4**, avec ou sans présence d'un ciment de liaison, comme cela se pratique habituellement dans les implantations de prothèses du type à queue. La fixation du plateau **30** peut aussi faire intervenir la présence de vis montées à travers des trous **33**.

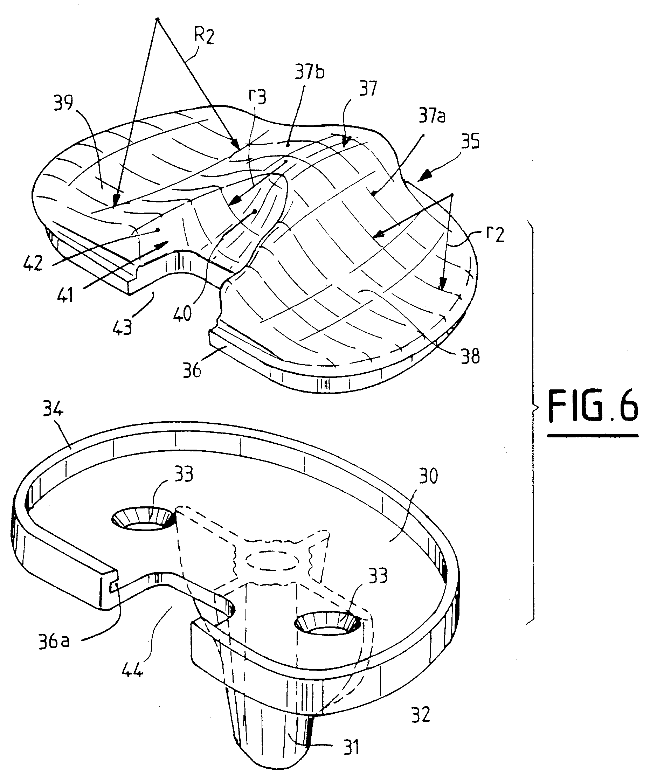

Le plateau tibial **30** est pourvu d'un bord périphérique **34** destiné à permettre le montage et l'immobilisation d'un patin **35** illustré, plus précisément, à la **fig. 6**. Le patin **35** est, de préférence, réalisé en une matière plastique appropriée, telle qu'en polyéthylène, alors que le plateau **30** est réalisé en un alliage métallique, semblable ou identique à celui constitutif de l'élément fémoral **1**. Le patin **35** est, de préférence mais non exclusivement, monté dans le plateau **30** par emboîtement-encliquetage et, à cette fin, comporte, préférentiellement, sur son bord arrière, une nervure **36** destinée à être encliquetée dans une rainure **36a** présentée par la partie postérieure du rebord **34**.

Le patin **35** est conformé pour offrir, sur son dessus, une épine **37** d'orientation antéro-postérieure, de part et d'autre de laquelle sont formées deux cavités glénoïdes **38** et **39**, respectivement dites interne et externe. Chaque cavité glénoïde, destinée à coopérer en tant que siège d'appui avec le condyle correspondant, est définie par un rayon de courbure **R**_{**2**} parallèle au plan sagittal largement supérieur au rayon de courbure **R** et par un rayon de courbure transversal **r**_{**2**} qui est lui aussi plus grand que le rayon **r** des condyles **13** et **14**.

L'épine **37** se développe à partir de la face antérieure du patin **35** en direction de la partie arrière et se raccorde à une cavité **40** qui est ménagée en creux à partir du sommet de l'épine **37**. La cavité **40** est définie, dans le plan sagittal, par un rayon de courbure **R**_{**3**} qui est plus grand que le rayon **R**_{**1**} et par un rayon de courbure transversal **r**_{**3**} qui est supérieur à celui de la section transversale semi-torique du corps **20**. La cavité **40** présente ainsi une forme de concavité peudo-hémisphérique qui fournit un logement ouvert de coopération avec la tête **19**. La cavité **40** s'ouvre vers l'arrière et se raccorde à un sillon **41** à flancs **42** divergents qui aboutissent au bord transversal arrière du patin **37**. De préférence, le sillon **41** présente, dans son fond et à partir du bord transversal arrière du patin **35**, une encoche **43** correspondant à une découpe **44** ménagée dans le plateau **30**.

La **fig. 1** montre que, dans une position sensiblement d'alignement entre le tibia et le fémur, les éléments fémoral et tibial de la prothèse, implantés comme il est dit sur les épiphyses **3** et **4**, coopèrent entre eux par l'intermédiaire des condyles **13** et **14** et des cavités glénoïdes **38** et **39**. Dans cette position, l'éminence **37** est engagée dans l'espace intercondylien, sensiblement à la naissance des condyles **13** et **14**, en considération de leur raccordement aux surfaces latérales convexes **10** et **11**. Une telle coopération assure un guidage dans le plan antéro-postérieur, sans interdire une rotation relative du tibia par rapport au fémur, en raison de la conformation de l'épine **37** alignée sensiblement avec le plan antéro-postérieur et raccordée, à partir d'un sommet arrondi qu'elle comporte par des pentes douces **37a** et **37b**, aux cavités glénoïdes **38** et **39**.

Dans cet état, l'éminence **18** est située à l'arrière de l'articulation en position surélevée par rapport au plateau tibial et ne coopère aucunement avec la cavité **40** ou le sillon **41**.

Une flexion du tibia, dans le sens de la flèche **f**_{**1**} ne modifie pas le rapport de coopération des surfaces jusqu'à une amplitude angulaire telle que, selon la **fig. 7**, la tête **19** est amenée à coopérer avec la cavité **40**. Il s'établit alors une butée relative assurant une postéro- stabilisation entre les éléments de la prothèse, de manière à interdire un glissement relatif antérieur du fémur produisant, tel que cela est connu, un effet de tiroir en l'absence des ligaments croisés.

En raison de la différence entre les rayons **R**_{**1**} et **R**_{**2**}, **r** et **r**_{**2**} et **r**_{**1**} et **r**_{**3**}, une telle position de butée postérostabilisatrice, interdisant un glissement antérieur du fémur, peut s'apprécier ou être assimilée à une sorte d'articulation à rotule qui présente l'avantage d'autoriser néanmoins, une rotation relative du tibia par rapport au fémur et de favoriser ainsi un fonctionnement anatomique proche, sinon identique, à celui autorisé naturellement par la conformation des surfaces coopérantes du genou et la présence des ligaments croisés et latéraux qui sont responsables du maintien de cette coopération de surface.

Si la flexion s'accroît, le corps **20** est progressivement engagé à l'intérieur du sillon **41** pour coopérer, par sa génératrice extérieure, avec le fond de la cavité **40**, tout en étant maintenu à distance des flancs divergents **42** qui, autorisent ainsi, également, une rotation relative du tibia par rapport au fémur.

Lorsqu'une flexion maximale, par exemple de l'ordre de 110^{o}, est atteinte, le corps semi-torique **20** est amené sensiblement en engagement partiel à l'intérieur de l'encoche **43**.

La rotation relative du tibia par rapport au fémur est également permise par la conformation des cavités glénoïdes **38** et **39** relativement aux condyles **13** et **14** correspondants, ainsi que par la forme divergente et asymétrique de ces derniers.

Ainsi, contrairement aux prothèses connues, du type à postéro-stabilisation en l'absence de ligaments croisés, la prothèse selon l'invention est à même de remplir les mêmes fonctions, tout en autorisant une rotation relative du tibia par rapport au fémur, pour favoriser une rotation interne ou externe, anatomique et physiologique, évitant de soumettre l'élément fémoral et l'élément tibial à des contraintes alternées susceptibles de provoquer un désordre d'ancrage et de fixation.

La rotation relative autorisée par la prothèse conforme à l'invention est, en outre, de nature à entretenir un fonctionnement plus naturel des ligaments subsistant et, notamment, des ligaments latéraux internes et externes.

## Revendications

1. Prothèse totale postéro-stabilisée du genou, comprenant :
- un élément fémoral (1) formant :
. deux surfaces latérales (10, 11) antérieures délimitant entre elles une trochlée fémorale (12),
. deux condyles (13, 14) faisant suite aux surfaces latérales se développant vers l'arrière et définissant un espace intercondylien (15) prolongeant la trochlée, sous la forme d'un voile intercondylien (16)
. et une butée de postéro-stabilisation, orientée vers l'avant et occupant en partie l'espace intercondylien
- et un élément titial (2) comprenant un plateau (30) d'adaptation et de support et un patin (35) dont la surface supérieure offre, pour la coopération avec l'élément fémoral, deux cavités glénoïdes (38 et 39) et une saillie médiane (37) définissant, vers l'arrière, une surface d'appui pour la butée;
caractérisée en ce que
- la butée est constituée par une éminence (18) qui est formée en saillie et qui se développe dans le plan sagittal vers l'arrière à partir du bord postérieur d'une fenêtre (17) ménagée dans le voile intercondylien (16), cette éminence étant constituée par un corps sensiblement semi-torique formant, à partir du bord postérieur de la fenêtre, une tête sensiblement semi-sphérique
- et la surface d'appui est définie par une cavité ménagée dans l'épine de manière à être ouverte vers l'arrière et à présenter une pseudo concavité de coopération avec la tête, une telle concavité étant définie
. par un rayon de courbure (R₃) dans le plan sagittal supérieur à celui du corps semi-torique
. et par un rayon de courbure transversale (r₃) supérieur à celui de la section transversale dudit corps
- et cette cavité se raccordant à un sillon arrière (41) à flancs (42) divergents.

2. Prothèse selon la revendication 1, caractérisée en ce que les cavités glénoïdes offrent des surfaces d'appui creuses définies, parallèlement au plan sagittal, par une courbure (**R**_{**2**}) de plus grand rayon que celui (**R**) des condyles et perpendiculairement audit plan, par une courbure (**r**_{**2**}) de plus grand rayon que celui (**r**) transversal de convexité des condyles.

3. Prothèse selon la revendication 1 ou 2, caractérisée en ce que les cavités glénoïdes sont sensiblement parallèles entre elles, alors que les condyles sont convergents en direction des surfaces latérales.

4. Prothèse selon la revendication 1, caractérisée en ce que l'espace intercondylien (**15**) est ouvert, dans sa partie comprise entre la tête de la butée et la base de la trochlée.

5. Prothèse selon la revendication 1, caractérisée en ce que la saillie (**18**) est ménagée pour que la tête (**19**) vienne coopérer en appui contre la cavité dans un état de flexion.

6. Prothèse selon la revendication 1 ou 5, caractérisée en ce que la cavité (**18**) aboutit à un sillon (**41**) à bords (**42**) divergents s'ouvrant sur le bord postérieur du patin (**35**).

7. Prothèse selon la revendication 1 ou 5, caractérisée en ce que la saillie (**18**) s'étend, au moins pour partie, extérieurement à l'enveloppe d'encombrement des condyles.

8. Prothèse selon la revendication 1, caractérisée en ce que le patin (**35**) est monté dans le plateau par emboîtement et encliquetage d'une nervure (**36**) dans une rainure (**36a**) interne d'un bord périphérique (**34**) du plateau.

## Claims

1. A postero-stabilized total prosthesis for the knee, comprising:
• a femur element (1) forming:
- two anterior lateral surfaces (10, 11) defining between them a femur trochlea (12);
- two condyles (13, 14) following on from the lateral surfaces and extending backwards to define an intercondyle gap (15) extending the trochlea; and
- a postero-stabilization abutment, oriented towards the front and occupying a portion of the intercondyle gap; and
• a tibial element (2) comprising a fitting-and-supporting tray (30) and a pad (35) whose top surface offers, for co-operation with the femur element, two glenoid cavities (38 and 39) and a middle projection (37) defining, towards the back, a bearing surface for the abutment;
• the abutment being constituted by a projection (18) projecting from the back portion of an intercondyle web (16) and including an anterior head (19) extended towards the back by an elongate body (20) substantially in the form of a semi-toroidal segment that is developed substantially in the sagittal plane (P-P') of the element; and
• the bearing surface being defined by a cavity (40):
- formed from the top of a spine (37) separating the glenoid cavities from the anterior edge of the pad;
- defined by a radius of curvature (R₃) in the sagittal plane greater than the radius of curvature of the semi-toroidal segment; and
- opening towards the back and connecting to a ferrule (41) having diverging flanks (42) terminating at the rear edge of the pad, the prosthesis being characterized by a transverse radius of curvature (r₃) of said cavity (40) that is greater than the radius of curvature of the cross-section of said segment.

2. A prosthesis according to claim 1, characterized in that the glenoid cavities provide hollow bearing surfaces defined parallel to the sagittal plane by curvature (R₂) of radius greater than the radius (R) of the condyles, and perpendicularly to said plane, by curvature (r₂) of radius greater than the transverse radius (r) of the convex shape of the condyles.

3. A prosthesis according to claim 1 or 2, characterized in that the glenoid cavities are substantially parallel to each other, while the condyles converge towards the lateral surfaces.

4. A prosthesis according to claim 1, characterized in that the intercondyle gap (15) is open in its portion between the head of the abutment and the bottom of the trochlea.

5. A prosthesis according to claim 1, characterized in that the projection (18) is organized so that the head (19) co-operates in abutment against the cavity when the prosthesis in the flexed state.

6. A prosthesis according to claim 1 or 5, characterized in that the cavity (18) reaches a ferrule (41) having diverging sides (42) opening out to the posterior edge of the pad (35).

7. A prosthesis according to claim 1 or 5, characterized in that the projection (18) extends at least in part outside the envelope occupied by the condyles.

8. A prosthesis according to claim 1, characterized in that the pad (35) is mounted in the tray by engagement and snap-fastening of a rib (36) in an internal groove (36a) of a peripheral rim (34) of the tray.

## Patentansprüche

1. Rückwärtig stabilisierte Knie-Gesamtprothese, mit:
- einem femoralen Element (1), das die folgenden Merkmale aufweist:
- zwei vordere Seitenflächen (10, 11), die zwischeneinander eine femorale Trochlea (12) begrenzen,
- zwei Kondylen (13, 14), die auf die seitlichen Flächen nachfolgen, sich nach hinten abwickeln und einen Kondylen-Zwischenraum (15) begrenzen, der die Trochlea verlängert, und
- einen Anschlag für die rückwärtige Stabilisierung, der nach vorne ausgerichtet ist und teilweise den Kondylen-Zwischenraum einnimmt, sowie
- einem tibialen Element (2), das eine Anpaß- und Trägerplatte (30) sowie einen Auflagekörper (35) aufweist, dessen obere Fläche zur Zusammenwirkung mit dem femoralen Element zwei Gelenkgruben (38 und 39) und einen mittigen Vorsprung (37) aufweist, der nach hinten eine Anlagefläche für den Anschlag bildet,
wobei
- der Anschlag von einer Erhöhung (18) gebildet ist, die vorspringend durch den hinteren Abschnitt eines Kondylen-Zwischenvelums (16) gebildet ist und einen vorderen Kopf (19) aufweist, der nach hinten durch einen länglichen Körper (20) im wesentlichen in der Form eines halb-torusförmigen Segments verlängert ist, das sich im wesentlichen in der sagittalen Ebene (P-P') des Elementes entwickelt, und
- die Anlagefläche von einer Höhlung (40) gebildet ist, die:
- vom Scheitel eines Grates (37) ausgehend eingebracht ist, der die Gelenkgruben zum vorderen Rand des Auflagekörpers hin trennt,
- durch einen Krümmungsradius (R₃) in der Sagittalebene über dem des halb-torusförmigen Segments definiert ist, und
- sich nach hinten öffnet und mit einer Furche (41) mit divergierenden Flanken (42) verbunden ist, die in den hinteren Rand des Auflagekörpers mündet,
gekennzeichnet durch einen querverlaufenden Krümmungsradius (r₃) der genannten Höhlung (40) über dem Querschnitt des genannten Segments.

2. Prothese nach Anspruch 1, dadurch gezeichnet, daß die Gelenkgruben definierte, hohle Auflageflächen parallel zur Sagittalebene durch eine Krümmung (R₂) mit größerem Radius als der (R) der Kondylen und senkrecht zur genannten Ebene durch eine Krümmung (r₂) mit größerem Radius als der querverlaufende (r) der Konvexität der Kondylen aufweisen.

3. Prothese nach Anspruch 1 oder 2, dadurch gekennzeichnet, daß die Gelenkgruben zueinander im wesentlichen parallel sind, während die Kondylen in Richtung der Seitenflächen konvergieren.

4. Prothese nach Anspruch 1, dadurch gekennzeichnet, daß der Kondylen-Zwischenraum (15) in seinem Abschnitt, der zwischen dem Anschlagkopf und der Basis der Trochlea liegt, offen ist.

5. Prothese nach Anspruch 1, dadurch gekennzeichnet, daß der Vorsprung (18) so eingebracht ist, daß im Zustand der Beugung der Kopf (19) zur Anlage gegen die Höhlung gelangt.

6. Prothese nach Anspruch 1 oder 5, dadurch gekennzeichnet, daß die Höhlung (18) in eine Furche (41) mit divergierenden Rändern (42) einmündet, die sich am hinteren Rand des Auflagekörpers (35) öffnet.

7. Prothese nach Anspruch 1 oder 5, dadurch gekennzeichnet, daß sich der Vorsprung (18) mindestens teilweise außerhalb der Umhüllenden der Abmessungen der Kondylen erstreckt.

8. Prothese nach Anspruch 1, dadurch gekennzeichnet, daß der Auflagekörper (35) auf der Platte durch Einsetzen und Einrasten einer Rippe (36) in eine Innennut (36a) eines Umfangsrandes (34) der Platte montiert ist.
